# EUROPEAN PATENT APPLICATION

(11) **EP 2 733 148 A1**
(43) Date of publication of application: **21.05.2014**
(21) Application number: 12192759.4
(22) Date of filing: 15.11.2012
(51) Int. Cl.: C07F 11/00, A61K 49/04

(54) **Tungsten complexes with di-dentate ligands**

(71) Applicant: Bayer Pharma Aktiengesellschaft, 13353 Berlin (DE)
(72) Inventor: Schmitt-Willich, Heribert, 10551 Berlin (DE); Berger, Markus, 12167 Berlin (DE)
(74) Representative: BIP Patents

(57) **Abstract**

The present invention describes a new class of highly stable W₃O₂ Clusters, a method for their preparation and their use as X-ray contrast agents.

## Description

The present invention describes a new class of trinuclear W₃O₂ tungsten clusters comprising bidentate di-, tri- or tetracarboxylic acid ligands, a method for their preparation and their use as X-ray contrast agents.

### Background of the invention

Synthesis and characterization of bi-oxo capped trinuclear tungsten clusters have been described by *de novo* synthesis starting from Na₂WO₄ (Powell, G.; Richens, D.T. Inorg.Chem. 1993, 32, 4021-4029 or W(CO)₆ (Bino, A.; Cotton, F. A.; Dori, Z.; Koch, S.; Kueppers, H.; Millar, M.; Sekutowski, J. C. Inorg. Chem. 1978, 17, 3245-3253, Bino, A.; Hesse; K. F.; Kueppers, H. Acta Crystallogr. 1980, B36, 723-725, Cotton, F.A.; Dori, Z.; Marler, D.O.; Schwotzer, W. Inorg.Chem 1984, 23, 4033-4038 and Cotton, F.A.; Dori, Z.; Marler, D.O.; Schwotzer, W. Inorg.Chem 1984, 23, 4738-4742) or by ligand exchange synthesis (WO 97/03993 and WO 97/03994) starting from preformed clusters. These tungsten clusters are part of the larger class of trinuclear bicapped clusters. The existence of this class is known for more than three decades and continuous research on their synthetic accessibility or behaviour and their general properties has been performed since then. Their general geometry was described by Powell, G.; Richens, D.T. Inorg.Chem. 1993, 32, 4021- 4029.

The water solubility and ability to absorb X-rays of these tungsten clusters and their potential use in diagnostic X-ray imaging has been described in WO 91/14460, WO 92/00698, WO 97/03993 and WO 97/03994.

Further investigations of these known tungsten clusters disclosed their chemical and physiological instability. Tungsten clusters with insufficient stability in aqueous solution or in the physiological environment of a patient would lead to a release of the ligand in the form of its free acid and the bare tungstate core. This would cause inacceptable toxic side effects in a patient, especially with respect to the expected high dose which will be necessary for a single diagnostic procedure. It is known from the well established triiodinated X-ray contrast agents that the dose for a single diagnostic procedure is in the range of 100-1500 mg iodine / kg body weight, equivalent to about 15-225 g of contrast agent for a 75 kg patient. Contrast media solutions are injected intravenously or intraarterially as a rapid bolus (usually in computed tomography (CT) via power injector) with the injection rate depending on the clinical indication or the examination protocol and area of interest.

Tungsten is characterized by a higher absorption coefficient for X-rays than iodine, especially in the range of tube voltages normally used in modern CT. A modern CT X-ray-tube, however, requires a minimum voltage of about 70 kV and reaches maximum voltage of 160 kV. As future technical developments in CT will not substantially change these parameters, iodine generally does not provide ideal attenuation features for this technology. In comparison to iodine the attenuation optimum (k-edge) of tungsten corresponds better to the ranges of voltages used in CT. Therefore the new tungsten clusters require a similar or lower contrast media dosage than conventional triiodinated contrast agents.

The use of tungsten based contrast agents will allow more flexibility for CT scanning protocols and lead to scan protocols that provide equivalent diagnostic value at lower radiation doses. Especially this feature is of high importance for CT. As technical development goals in terms of spatial and temporal resolution have approached the limit of clinical significance, reduction of the radiation burden of CT scanning has today become a central aspect of the development of new CT scanners and x-ray machines. Following the widely accepted ALARA-rule (radiation exposure has to be reduced to levels: As Low As Reasonably Achievable), the new tungsten based contrast agents will contribute to high-quality diagnostic imaging at reduced radiation exposure.

The aim of the present invention was to provide sufficiently stable, water soluble and well tolerated tungsten clusters for use as X-ray contrast agent in diagnostic imaging, especially in modern computed tomography.

This aim was achieved by the provision of the compounds of the present invention. Surprisingly, it was observed that monodentate ligands claimed in WO 97/03993 and WO 97/03994 can be replaced by bidentate ligands and that the clusters described in this patent application show a highly increased stability under heat sterilization conditions of the aqueous solution, and have an excellent tolerability in experimental animals as well as a high *in vivo* stability.

The invention of suitable new bidentate ligands for the W₃O₂ core and the synthesis of new stable tris bidentate W₃O₂ clusters enabled for the first time the practical use of this compound class as X-ray contrast agents in diagnostic imaging. By enabling tungsten based contrast agents the option to reduce radiation dose is a clear advantage of W₃O₂ clusters of the present invention over existing iodine based contrast agents due to the higher absorption coefficient of tungsten for X-rays compared to iodine.

### Summary of the invention

The present invention describes a new class of highly stable W₃O₂ clusters, a method for their preparation and their use as X-ray contrast agents.

### Detailed Description of the invention

In a first aspect, the present invention is directed to trinuclear tungsten clusters comprising bidentate carboxylic acid ligands, especially comprising at least two 3-propionic acid structure elements in each ligand.

In a preferred embodiment the trinuclear tungsten cluster comprises a W₃O₂ core.

In another preferred embodiment the trinuclear tungsten cluster comprises three di-, tri- or tetracarboxylic acid ligands.

In a second aspect, the present invention is directed to compounds of the general formula I, 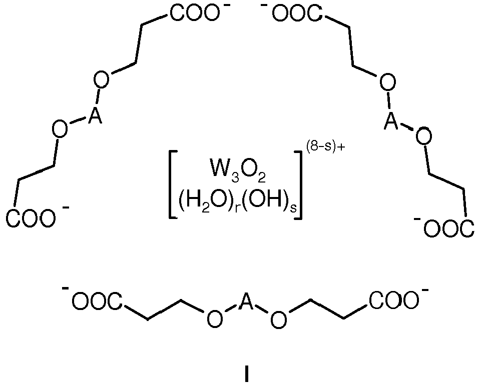 wherein
A is (CH₂)₂, (CH₂)₃, (CH₂)₄, (CH₂)₂-O-(CH₂)₂, CH₂-X-CH₂, (CH₂)₂-X-(CH₂)₂;
wherein X is CHOH, CHNH₂, C(CH₂OH)₂, C(CH₂OCH₃)₂, C(CH₂O(CH₂)₂COOH)₂, C(CH₂H)(CH₂O(CH₂)₂COOH), C(CH₂CH₃)(CH₂O(CH₂)₂COOH), CHNHCH₂COOH, CHNHCH₂CH₂OH, CHNH(CH₂)₂COOH, CHCH₂COOH, CH(CH₂)₂COOH;
r is 0, 1, 2, 3;
and
sis0,1,2,3;
with the proviso that r + s is 3;
if necessary including any protonated species and any deprotonated species of said compounds, including all isomeric forms of said compounds, including but not limited to enantiomers, diastereomers, regioisomers and mixtures thereof, and any pharmaceutically acceptable salt of such compounds.

As known to the person skilled in the art, the central tris aqua W₃O₂ cluster ion acts as a polyprotic acid in aqueous solution. Dependent on the pH of the solution different protonation species [W₃O₂(H₂O)₃]⁸⁺, [W₃O₂(H₂O)₂(OH)]⁷⁺, [W₃O₂(H₂O)(OH)₂]⁶⁺ and [W₃O₂(OH)₃]⁵⁺ are present (Bino, A.; Gibson, D. Inorganica Chimica Acta, 1985, 104, 155-160). Additional counter ions may be present.

Trinuclear tungsten clusters of the general formulae I, which are charged at physiological pH, can be neutralized by addition of suitable, physiologically biocompatible counter ions, e.g. sodium ions or suitable cations of organic bases including, among others, those of primary, secondary or tertiary amines, for example *N*-methylglucamine. Lysine, arginine or ornithine are suitable cations of amino acids, as generally are those of other basic naturally occurring amino acids.

Suitable anions are the anions of acids, inorganic acids, such as, for example, hydrochloric acid, phosphoric acid and sulfuric acid, as well as the anions of organic acids such as, for example, acetic acid, citric acid, aspartic acid, glutamic acid, among others can be used.

A preferred compound of the general formula I is: Monoaqua-κ*O*-bis{µ₃-3,3'-[ethanediylbis(oxy)]dipropanoato-1κ*O*¹:2κ²*O*^{1'},*O*²:3κ*O*^{2'}}{µ-3,3'-[ethanediylbis(oxy)]dipropanoato}1κ²*O*¹,*O*²-:2κ²*O*^{1'}, *O*^{2'}-dihydroxido-κ²*O*-di-µ3-oxido-1:2:3κ⁶*O*-triangulo-tritungsten(IV)(3W-W)

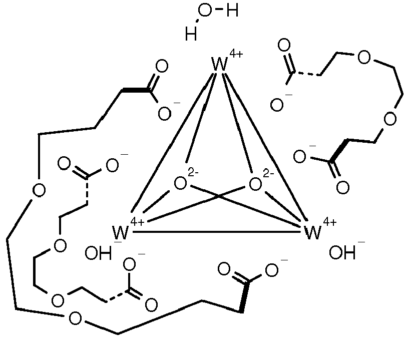

Another preferred compound of the general formula I is:
Monoaqua-κ*O*-tris{µ-3,3'-[ethanediylbis(oxy)]dipropanoato]-1κ⁴*O*¹,O²,*O*³,*O*⁴:2κ⁴*O*^{1'},*O*^{2'},*O*⁵,*O*⁶:3κ⁴*O*^{3'},*O*^{4'},*O*^{5'},*O*^{6'}-dihydroxido-κ²-*O*-di-µ₃-oxido-1:2:3κ⁶*O-*triangulo-tritungsten(IV)(3 W-W)

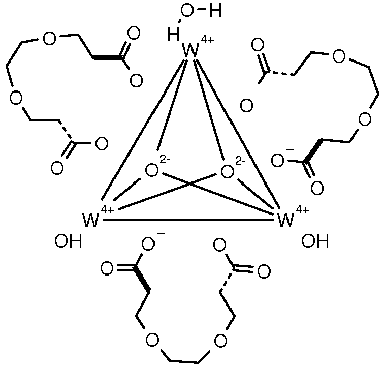

Another preferred compound of the general formula I is:
Monoaqua-κ*O*-bis{µ₃-3,3'-[propane-1,3-diylbis(oxy)]dipropanoato-1κ*O*¹:2κ²*O*^{1'},*O*²:3κ*O*^{2'}}{µ-3,3'-[propane-1,3-diylbis(oxy)]dipropanoato}1κ²*O*¹,*O*²:2κ²*O*^{1'},O^{2'}-dihydroxido-κ²*O*-di-µ₃-oxido-1:2:3κ⁶*O-triangulo*-tritungsten(IV)(3 W-W)

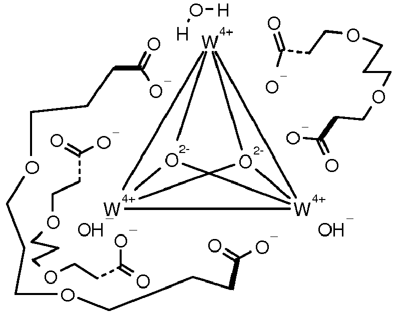

Another preferred compound of the general formula I is:
Monoaqua-κ*O*-tris(µ-3,3'-{oxybis[2,1-ethanediylbis(oxy)]}dipropanoato)1κ⁴*O*¹,*O*²,*O*³,*O*¹: 2κ⁴*O*^{1'},*O*^{2'},*O*⁵,*O*⁶:3κ⁴*O*^{3'},*O*^{4'},*O*^{5'},*O*^{6'}-dihydroxido-κ²*O*-di-µ₃-oxido-1:2:3κ⁶*O*-triangulo-tritungsten(IV)(3 W-W)

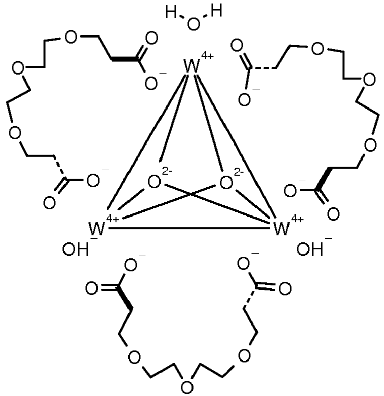

Another preferred compound of the general formula I is:
Monoaqua-κ*O*-bis(µ₃-3,3'-{oxybis[2,1-ethanediylbis(oxy)]}dipropanoato-1κ*O*^{1'}:2κ²*O*²,*O*²: 3κ*O*^{2'})(µ-3,3'-{oxybis[2,1-ethanediylbis(oxy)]}dipropanoato)1κ²*O*¹,*O*²:2κ²*O*¹,*O*^{2'}-dihydroxido-κ²*O*-di-µ₃-oxido-1:2:3κ⁶*O*-triangulo-tritungsten(IV)(3 W-W)

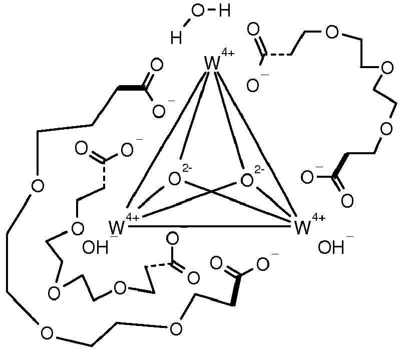

Another preferred compound of the general formula I is:
Monoaqua-κ*O*-tris{µ₂ -3,3'-[1,3-bis(2-carboxyethoxy)propan-2,2-diyl]bismethyl (oxy)dipropanoato}1κ⁴*O*¹,*O*²,*O*³,*O*⁴:2κ⁴*O*^{1'},*O*^{2'},*O*⁵,*O*⁶:3κ²,*O*^{3'},*O*^{4'},*O*^{5'},*O*⁶dihydroxido-κ²*O*-di-µ₃-oxido-1:2:3x⁶*O*-*triangulo*-tritungsten(IV)(3 W-W)

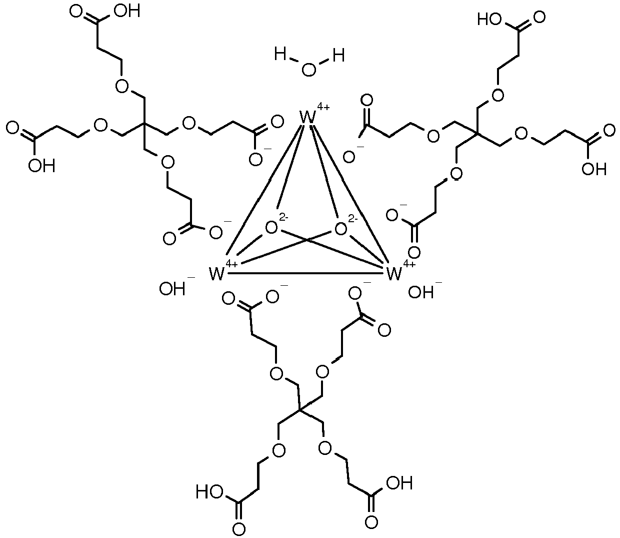

Another preferred compound of the general formula I is:
Monoaqua-κ*O*-trislp-3,3'-[1-(2-carboxyethoxy)-3-methoxypropan-2,2-diyl]bismethyl(oxy) dipropanoato}1κ⁴*O*¹,*O*²,*O*³,*O*⁴:2κ⁴*O*^{1'},*O*^{2'},*O*⁵,*O*⁶:3κ²*O*^{3'},O^{4'},O^{5'},O^{6'}-dihydroxido-κ²-di-µ₃-oxido-1:2:3κ⁶*O*-triangulo-tritungsten(IV)(3 W-W)

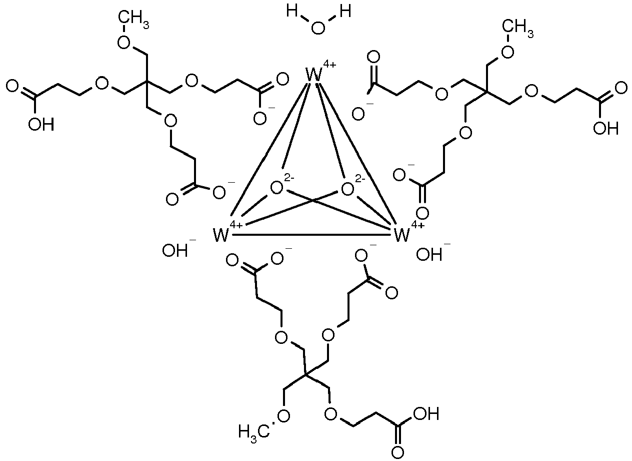

Another preferred compound of the general formula I is:
Monoaqua-κ*O*-tris{µ-3,3'-[1,3-dihydroxypropan-2,2-diyl]bismethyl(oxy)dipropanoato} 1K⁴*O*¹,*O*²*O*³,*O*⁴:2κ⁴*O*^{1'},*O*^{2'},*O*⁵*,O*⁶:3κ²O^{3'},*O*^{4'},*O*⁵,*O*^{6'}-dihydroxido-κ²*O*-di-µ₃-oxido-1:2:3κ⁶*O-*triangulo-tritungsten(IV)(3 W-W)

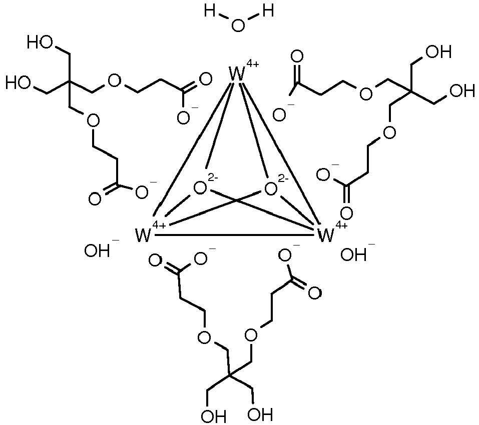

Another preferred compound of the general formula I is:
Monoaqua-κ*O*,-tris{µ-3,3'-[propane-1,3-diylbis(oxy)]dipropanoato} 1κ⁴*O*¹,*O*²,*O*³,*O*⁴: 2κ⁴*O*^{1'},*O*²,*O*³,*O*⁵,*O*⁶:3κ⁴*O*^{3'},*O*^{4'},*O*⁵,*O*^{6'}-dihydroxido-κ²*O*-di-µ₃-oxido-1:2:3κ⁶*O*-*triangulo-*tritungsten(IV)(3 W-W)

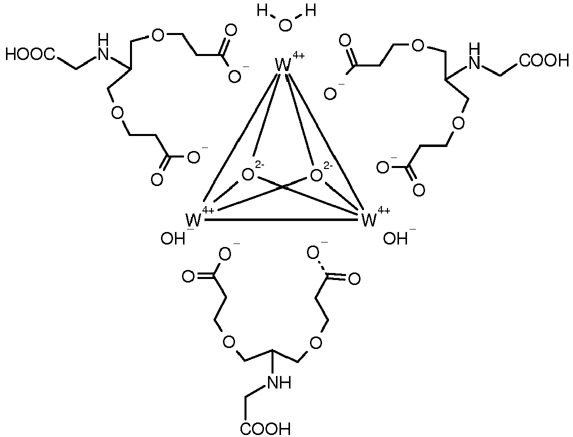

In a third aspect, the invention is directed to the use of compounds of the general formulae 15II as intermediate for the preparation of trinuclear tungsten clusters comprising bidentate carboxylic acid ligands, 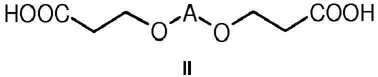 wherein
A is (CH₂)₂, (CH₂)₃, (CH₂)₄, (CH₂)₂-O-(CH₂)₂,CH₂-X-CH₂, (CH₂)₂-X-(CH₂)₂; wherein X is CHOH, CHNH₂, C(CH₂OH)₂, C(CH₂OH₃)₂, C(CH₂O(CH₂)₂COOH)₂,C(CH₂OH)(CH₂O(CH₂)₂COOH), C(CH₂OCH₃)(CH₂O(CH₂)₂COOH), CHNHCH₂COOH, CHNHCH₂CH₂OH, CHNH(CH₂)₂COOH;

In a preferred embodiment the invention is directed to the use of compounds of the general formulae II for the manufacture of the compounds of the general formula I.

The Process for the preparation of trinuclear tungsten clusters comprising three bidentate carboxylic acid ligands of general formula I contains the reaction of monoaqua-κ*O-*hexakis(µ-acetato-κ²*O*)-dihydroxido-K²*O*-di-µ₃-oxido-1:2:3κ⁶*O*-*triangulo*-tritungsten(3 *W-W*)(IV) or a salt of this cluster or sodium hexakis(µ-acetato-κ²*O*)-tris(acetato-κ*O*)-di-µ₃-oxido-1:2:3κ⁶*O*-*triangulo*-tritungsten(3 *W-W*)(IV) in aqueous solution with three aliquots or slight excess of the desired bidentate ligands (compound of the general formulae II), heating the components to temperatures in the range from 80° to 150°C in combination with the optional use of a pressure vessel, if necessary applying microwave irradiation, applying heating times from 10 minutes up to 3 days, isolation and purification of the resulting clusters by reversed phase and/or ion exchange chromatography.

In a fourth aspect, the invention is directed to the new trinuclear tungsten clusters comprising three bidentate carboxylic acid ligands of general formulae I, obtainable by ligand exchange reaction of monoaqua-κ*O*-hexakis(µ-acetato-κ²*O*)-dihydroxido-κ²*O*-di-µ₃-oxido-1:2:3κ⁶*O*-*triangulo*-tritungsten(3 *W-W*)(IV) or a salt of this cluster or sodium hexakis(µ-acetato-K²*O*)-tris(acetato-κ*O*)-di-µ₃-oxido-1:2:3κ⁶*O*-triangulo-tritungsten(3 *W-W*)(IV) in aqueous solution in the presence of three aliquots or slight excess of the desired bidentate ligand, heating the components to temperatures in the range from 80°to 150°C in combination with the optional use of a pressure vessel, if necessary applying microwave irradiation, applying heating times from 10 minutes up to 3 days, isolation and purification of the resulting clusters by reversed phase and/or ion exchange chromatography.

In a fifth aspect, the invention is directed to compounds of general formula I for the manufacture of diagnostic agents, especially of X-ray diagnostic agents for adminstration to humans or animals.

For the manufacture of diagnostic agents, for example the adminstration to human or animal subjects, the compounds of general formulae I will conveniently be formulated together with pharmaceutical carriers or excipient. The contrast media of the invention may conveniently contain pharmaceutical formulation aids, for example stabilizers, antioxidants, pH adjusting agents, flavors, and the like. They may be formulated for parenteral or enteral administration or for direct administration into body cavities. For example, parenteral formulations contain a steril solution or suspension in a concentration range from 150 to 600 mg W/mL, especially 200 to 450 mg W/mL of the new tris bidentate W₃O₂ clusters according to this invention. Thus the media of the invention may be in conventional pharmaceutical formulations such as solutions, suspensions, dispersions, syrups, etc. in physiologically acceptable carrier media, preferably in water for injections. When the contrast medium is formulated for parenteral administration, it will be preferably isotonic or hypertonic and close to pH 7.4.

Pharmaceutically acceptable salts of the compounds according to the invention also include salts of customary bases, such as, by way of example and by way of preference, alkali metal salts (for example sodium salts), alkaline earth metal salts (for example calcium salts) and ammonium salts, derived from ammonia or organic amines having 1 to 16 carbon atoms, such as, by way of example and by way of preference, N-methylglucamine.

For use as X-ray contrast agent, the media of the invention should generally have a sufficiently high percentage of tungsten, in particular a contrast medium with a high content of tungsten per molecule (minimum 35 % or higher).

### General synthesis of compounds of the invention

Bidentate carboxylic acids were synthesized via the bis nitrile in analogy to published procedures *(*Bul/.Chem.Soc.Jpn 1988, 61, 2443-2449) or via alkyl ester derived from alkyl acrylates in analogy to published procedures *(*Eur. J. Inorg. Chem. 2001, 1789-1795). Hydrolysis of bis nitriles was acomblishyd by of strong acids like hydrochloric acid or trifluoromethane sulfonic acid. Cleavage of alkyl ester was established by the use of strong acids like hydrochloric acid or trifluoromethane sulfonic acid, trifluoro acetic acid and formic acid. Basic saponification is also possible. Chemical transformations like reductive aminations, or amid formation at A are possible at the ester or nitrile form. 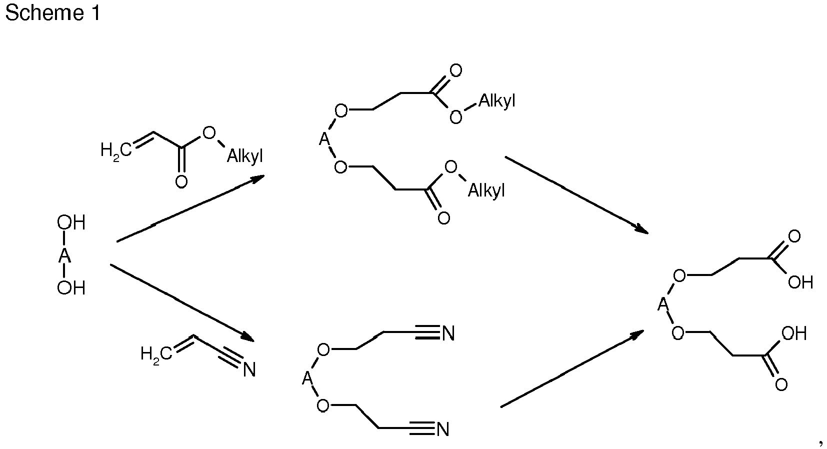 wherein A is as defined above

New W₃O₂ clusters were synthesized by ligand exchange reaction (WO 97/03994) of known tri-tungsten clusters like the hexa acetate or the nona acetate in aqueous solution in the presence of an three times aliquot or slight excess of the desired bisdentate ligand to obtain a new mixed W₃O₂ cluster. Heating temperatures generally range from 80° to 150°C in combination with the optional use of a pressure vessels. Microwave irradiation is a possible alternative to conventional heating sources. Heating times range from 10 minutes up to 3 days. Isolation and purification of the desired cluster is obtained by conventional chromatographic methods like preparative HPLC or ion exchange chromatography. 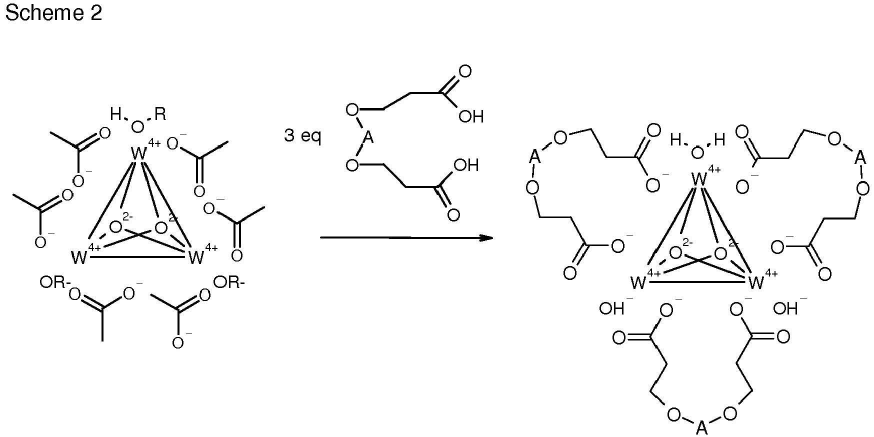 wherein A is as defined above

### Definitions

If chiral centres or other forms of isomeric centres are not otherwise defined in a compound according to the present invention, all forms of such stereoisomers, including enantiomers and diastereoisomers, are intended to be covered herein. Compounds containing chiral centres may be used as racemic mixture or as an enantiomerically enriched mixture or as a diastereomeric mixture or as a diastereomerically enriched mixture, or these isomeric mixtures may be separated using well-known techniques, and an individual stereoisomer maybe used alone.

Pharmaceutically acceptable salts of the compounds according to the invention include salts of mineral acids, carboxylic acids and sulfonic acids, for example salts of hydrochloric acid, sulfuric acid, phosphoric acid, methanesulfonic acid, ethanesulfonic acid, toluenesulfonic acid, benzenesulfonic acid, acetic acid, propionic acid, lactic acid, tartaric acid, malic acid, citric acid, fumaric acid, maleic acid and benzoic acid.

### Experimental Part

**Abbreviations**

| | |
|---|---|
| br | broad signal (in NMR data) |
| DAD | diode array detector |
| HPLC | high performance liquid chromatography |
| ICP-OES | Inductively coupled plasma - optical emission spectrometry |
| ICP-MS | Inductively coupled plasma - mass spectrometry |
| MS | mass spectrometry |
| m | multiplet |
| NMR | nuclear magnetic resonance spectroscopy: chemical shifts (δ) are given in ppm. |
| quin | quintett |
| rt | room temperature |
| s | singlet |
| t | triplet |
| td | triplet of doublet |
| THF | tetrahydrofuran |

### Examples

### Intermediate 1

### Sodium hexakis(µ-acetato-κ²O)-tris(acetato-κO)-di-µ3-oxido-1:2:3κ⁶O-triangulo-tritungsten(3 W-W)(IV)

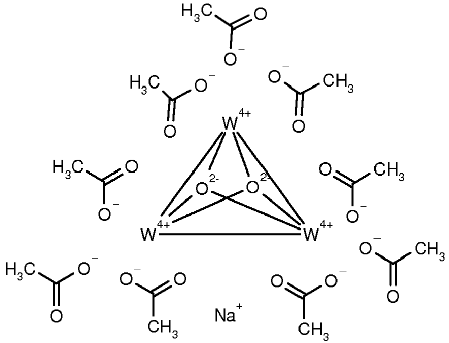

A suspension of sodium tungstate (26.4 g, 89.8 mmol) and tungsten hexacarbonyl (Aldrich, 97% purity, 130.4 g, 359.4 mmol) in acetic anhydride (2173 mL, 23.0 mol) was heated to 80°C. Air (approx. 5 L per minute) was bubbled through the mixture for 15 minutes, then the air stream was stopped and the bath temperature was raised to 145°C. After 18 hours, the mixture was cooled to rt. The yellow-brownish precipitate was filtered, washed with actic anhydride (100 mL), THF (200 mL) and *tert*-butylmethyl ether (200 mL) and dried (50°C, 50 mbar) to give sodium hexakis(µ-acetato-κ²*O*)-tris(acetato-κ*O*)-di-µ₃-oxido-1:2:3κ₆*O*-*triangulo*-tritungsten(3 *W-W*)(IV) (127.8g, 112.3 mmol, 93,7% based on tungsten hexacarbonyl).
**¹H-NMR** (300 MHz, deuterium oxide) δ = 2.08 (s, 9 H), 2.28 (s, 18 H).
**LC/MS ES-** m/z 1115.03 (M-23).

### Intermediate 2

### Monoaqua-κO-hexakis(µ-acetato-κ²O)-dihydroxido-κ² O-di-µ₃-oxido-1:2:3κ⁶O-triangulo-tritungsten(3 W-W(IV)

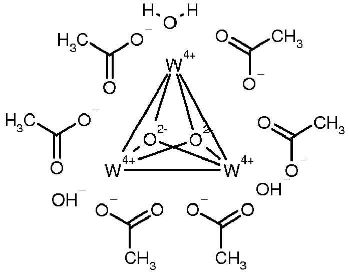

Sodium hexakis(µ-acetato-κ²*O*)-tris(acetato-κ*O*)-di-µ₃-oxido-1:2:3κ⁶*O*-*triangulo*-tritungsten-(3 *W-W*(IV) (20 g, 17.6 mmol) was refluxed in water (500mL) for 15 hours. The mixture was concentrated under vacuum and lyophilized to yield 18.3 g of raw monoaqua-κ*O-*hexakis(µ-acetato-κ²*O*)-dihydroxido-κ²*O*-di-µ₃-oxido-1:2:3κ⁶*O*-*triangulo*-tritungsten(3 *W-W*)(IV) together with sodium acetate and acetic acid.
**¹H-NMR** (300 MHz, deuterium oxide) δ = 2.25 (s, 18 H).
**LC/MS ES-** m/z = 989.3 (M-1).

### Example 1

### Monoaqua-κO-bis{µ₃-3,3'-[ethanediylbis(oxy)]dipropanoato-1κO¹:2κ²O^{1'},O²:3κO^{2'}}{µ-3,3'-[ethanediylbis(oxy)]dipropanoato}1κ²O¹,O²:2κ²O^{1'},O^{2'}dihydroxido-κ²O-di-µ3-oxido-1:2:3κ⁶O-triangulo-tritungsten(IV)(3 W-W)

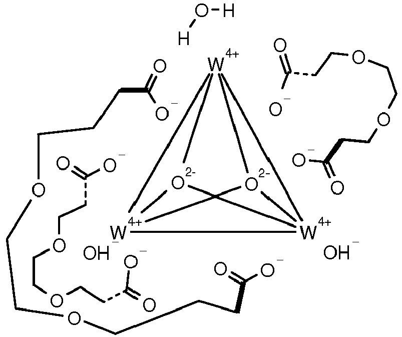

### Example 1 a

### 3,3'-[Ethane-1,2-diylbis(oxy)]dipropanoic acid

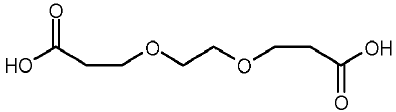

3,3'-[Ethane-1,2-diylbis(oxy)]dipropanoic acid was synthesized analogous to Ashikaga et al. Bull.Chem.Soc.Jpn 1988, 61, 2443-2449 via bis nitrile.
**¹H-NMR** (300 MHz, D₂O) δ = 2.62 (t, 4H), 3.63 (s, 4H), 3.75 (t, 4H) ppm.

### Example 1 b

### Monoaqua-κO-bis{µ₃-3,3'-[ethanediylbis(oxy)]dipropanoato-1κO¹:2κ²O^{1'},O²:3κO^{2'}}{µ-3,3'-[ethanediylbis(oxy)]dipropanoato}1κ²O¹,O²:2κ²O^{1'}, O^{2'}-dihydroxido-κ²O-di-µ₃-oxido-1:2:3κ⁶O-triangulo-tritungsten(IV)(3 W-W)

A suspension of 3,3'-[ethane-1,2-diylbis(oxy)]dipropanoic acid (250 mg, 1.21 mmol) and hexacis(µ-acetato-κ²*O*)-monoaqua-κ*O*-dihydroxido-κ²*O*-di-µ₃-oxido-1:2:3κ⁶*O*-*triangulo-*tritungsten(IV) (400 mg, 0.40 mmol) in acetic acid (93 µL, 1.62 mmol) and water (30 mL) was irradiated in a micowave reactor for 15 minutes at 140°C. The reaction mixtures were filtrated and the fitrates was concentrated in vacuum. Separation on a preparative HPLC (acetonitrile water + acetic acid) yields 48 mg monoaqua-κ*O*-bis{µ₃-3,3'-[ethanediylbis(oxy)]dipropanoato-1κ*O*¹:2κ²*O*^{1'}, *O²*:3κ*O*^{2'}}{µ-3,3'-[ethanediylbis(oxy)-dipropanoato}-1 κ²*O*²,*O*²:2κ²*O*^{1'},*O*^{2'}*-*dihydroxido-κ²*O-*di*-*µ₃*-*oxido*-*1:2:3κ⁶*O-triangu*/*o-*tritungsten(IV)(3 W-W) and 97 mg of example 2.
**¹H-NMR** (300 MHz, D₂O) δ = 2.65 - 2.80 (m, 8 H), 2.81 - 2.94 (m, 4 H), 3.42 - 3.54 (m, 4 H), 3.55 - 3.68 (m, 12 H), 3.84 (t, 4 H), 3.94 (td, 4 H) ppm.
**LC/MS ES-** m/z = 1247.12 (M-1).

### Example 2

### Monoaqua-κO-tris{µ-3,3'-[ethanediylbis(oxy)]dipropanoato}1κ⁴O¹,O²,O³,O³,O⁴: 2κ⁴O^{1'},O²,O⁵,O⁶:3κ⁴O^{3'},O^{4'},O^{5'},O^{6'}-dihydroxido-κ²O-di-µ₃-oxido-1:2:3κ⁶O-triangulo-tritungsten(IV)(3 W-W)

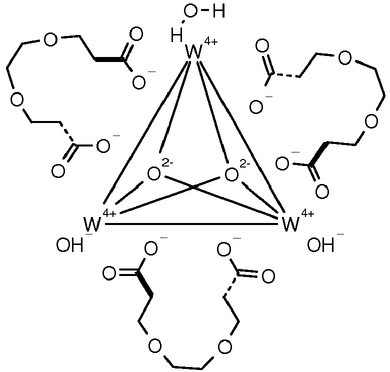

97 mg of the title compound were isolated as second fraction of example 1.
¹H NMR (300 MHz, D₂O) δ = 2.79 (t, 12 H), 3.61 (s, 12 H), 3.85 (t, 12 H) ppm.
**LC/MS ES-** m/z = 1246.86 (M-1).

### Example 3

### Monoaqua-κO-bis{µ₃-3,3'-[propane-1,3-diylbis(oxy)]dipropanoato-1 κO¹:2κ²O^{1'},O²: 3κO^{2'}}{µ-3,3'[propane.1,3-diylbis(oxy)]dipropanoato}1κ²O¹, O²:2κ²O^{1'},O^{2'}-dihydroxido-κ²O-di-µ3-oxido-1:2:3κ⁶O-triangulo-tritungsten(IV)(3 W-W)

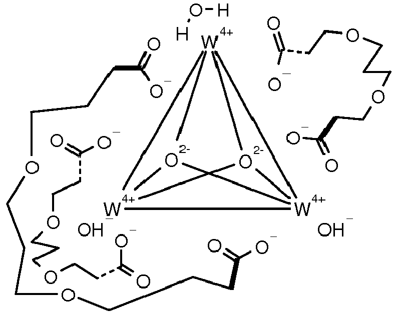

### Example 3a

### 3,3'-[Propane-1,2-diylbis(oxy)]dipropanoic acid

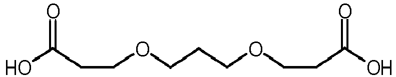

3,3'-[Propane-1,2-diylbis(oxy)]dipropanoic acid was synthesized analogous to Senkyr et al. Analytical Chemistry 1979, 51, 786 via bis nitrile.
**¹H-NMR** (400 MHz, D₂O) δ = 1.78 (quin, 2H), 2.60 (t, 4H), 3.54 (t, 4H), 3.71 (t, 4H) ppm.

### Example 3b

### Monoaqua-κO-bis{µ₃-3,3'-[propane-1,3-diylbis(oxy)]dipropanoato-1κO¹:2κ²O^{1'}, O²: 3κO^{2'}}{µ-3,3'-[propane-1,3-diylbis(oxy)]dipropanoato}1κ²O¹,O²:2κ²O¹,O^{2'}-dihydroxido-κ²Odi-µ₃-oxido-1:2:3κ⁶O-atriangulo-tritungsten(IV)(3 W-W)

A suspension of 3,3'-[propane-1,2-diylbis(oxy)]dipropanoic acid (267 mg, 1.21 mmol) and tritungsten(IV) (400 mg, 0.40 mmol) in acetic acid (93 µL, 1.62 mmol) and water (30 mL) was irradiated in a micowave reactor for 15 minutes at 140°C. The reaction mixtures were filtrated and the fitrates was concentrated in vacuum. Separation on a preparative HPLC (acetonitrile water + acetic acid) yields 30.6 mg monoaqua-κ*O*-bis{µ₃-3,3'-[propanediyibis(oxy)]dipropanoato-1κ*O*¹:2κ²*O*^{1'},*O*²:3κ*O*²}{u-3,3'-[propanediyibis(oxy)] dipropanoato}-1κ²*O*¹,*O*²:2κ²*O*¹,*O*^{2'}*-*dihydroxido*-*κ²*O-*di*-*µ₃*-*oxido*-*1:2:3κ⁶*O-triangulo-*tritungsten(IV)(3 W-W)
**¹H-NMR** (300 MHz, D₂O) δ = 1.58 - 1.68 (m, 4 H), 1.75 - 1.86 (m, 2 H), 2.63 - 2.86 (m, 12 H), 3.31 (t, 8 H), 3.54 (t, 4 H), 3.73 - 3.96 (m, 12 H) ppm.
**LC/MS ES-** m/z = 1289.21 (M-1).

### Example 4

### Monoaqua-κO-tris(µ-3,3'-{oxybis[2,1-ethanediylbis(oxy)]}dipropanoato) 1κ⁴O¹,O²,O³,O⁴:2κ⁴O^{1'},O⁵,O⁶:3κ⁴O^{3'},O^{4'},O^{5'},O^{6'}-dihydroxido-κ²O-di-µ3-oxido-1:2:3κ⁶O-triangulo-tritungsten(IV)(3 W-W)

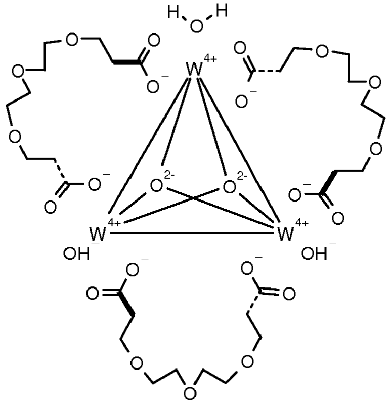

### Example 4a

### 3,3"[Oxybis(ethane-2,1-diyloxy)]dipropanoic acid

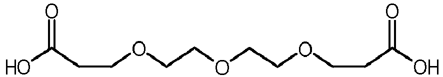

3,3'-[Oxybis(ethane-2,1-diyloxy)]dipropanoic acid was synthesized analogous to Ashikaga et al. Bull.Chem.Soc.Jpn 1988, 61, 2443-2449 via bis nitrile.
**¹H-NMR** (300 MHz, D₂O) δ = 2.64 (t, 4H), 3.64 (s, 8H), 3.76 (t, 4H) ppm.

### Example 4b

### Monoaqua-κO-tris(µ-3,3'-{oxybis[2,1-ethanediylbis(oxy)]}dipropanoato) 1κ⁴O¹,O²,O³,O⁴:2κO^{1'},O^{2'},O⁵,O⁶:3κ⁴O^{3'},O^{4'},O^{5'},O^{6'}-dihydroxido-κ²O-di-µ₃-oxido-1:2:3κ⁶O-triangulo-tritungsten(IV)(3 W-W)

A suspension of 3,3'-[oxybis(ethane-2,1-diyloxy)]dipropanoic acid (303 mg, 1.21 mmol) and hexacis(µ-acetato-κ²*O*)-monoaqua-κ²*O*)-dihydroxido-κ*O*-di-µ₃-oxido-1:2:3κ⁶*O-*triangulo-tritungsten(IV) (400 mg, 0.40 mmol) in water (30 mL) was irradiated in a micowave reactor for 15 minutes at 140°C. The reaction mixtures were filtrated and the fitrates was concentrated in vacuum. Separation on a preparative HPLC (acetonitrile water + acetic acid) yields 7 mg monoaqua-κ*O*-tris(µ-3,3'-{oxybis[2,1-ethanediylbis (oxy)]}dipropanoato)-1κ⁴*O*¹,*O*²,*O*⁴:2κ⁴*O*^{1'},*O*^{2'},*O*^{2'}, *O*⁵, *O*⁶:3κ⁴*O*^{3'},*O*^{4'},*O*^{5'},*O*^{6'}-dihydroxido-κ²*O-*di-µ₃-oxido-1:2:3κ⁶*O*-*triangulo*-tritungsten(IV)(3 W-W) and 37 mg of example 5.
**¹H-NMR** (300 MHz, D₂O) δ = 2.89 (t, 12 H), 3.62 - 3.69 (m, 12 H), 3.70 (m, 12 H), 3.88 (t, 12 H) ppm.
**LC/MS ES-** m/z = 1379.21 (M-1).

### Example 5

### Monoaqua-κO-bis(µ₃-3,3'-{oxybis[2,1-ethanediylbis(oxy)]}dipropanoato-1κO¹: 2κO^{1'},O²:3κO^{2'})(µ₃-3,3'-{oxybis[2,1-ethanediylbis(oxy)]}dipropanoato)1κ²O¹,O²: 2κ²O^{1'},O^{2'}-dihydroxido-κ²O-di-µ₃-oxido-1:2:3κ⁶O-triangulo-tritungsten(IV)(3 W-W)

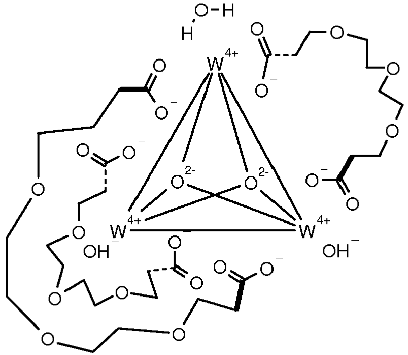

37 mg of the title compound were isolated as second fraction of example 4.
¹H NMR (400 MHz, D₂O) δ = 2.80 - 2.89 (m, 8 H), 2,91 - 2.96 (m, 4H), 3.49 - 3.56 (m, 8 H), 3.57 - 3.61 (m, 8 H), 3.66 - 3.70 (m, 4 H), 3.72 - 3.75 (m, 4 H), 3.83 - 3.92 (m, 12 H) ppm.
**LC/MS ES-** m/z = 1379.09 (M-1).

### Example 6

### Monoaqua-κO-tris{µ₂-3,3'-[1,3-bis(2-carboxyethoxy)propan-2,2-diyl]bismethyl (oxy)dipropanoato}1κ⁴O¹,O³,O⁴:2κ⁴O^{1'},O^{2'},O^{5'},O⁶:3κ²O^{3'},O^{4'},O^{5'},O^{6'}-dihydroxido-κ²O-di-µ₃-oxido-1:2:3κ⁶O-triangulo-tritungsten(IV)(3 W-W)

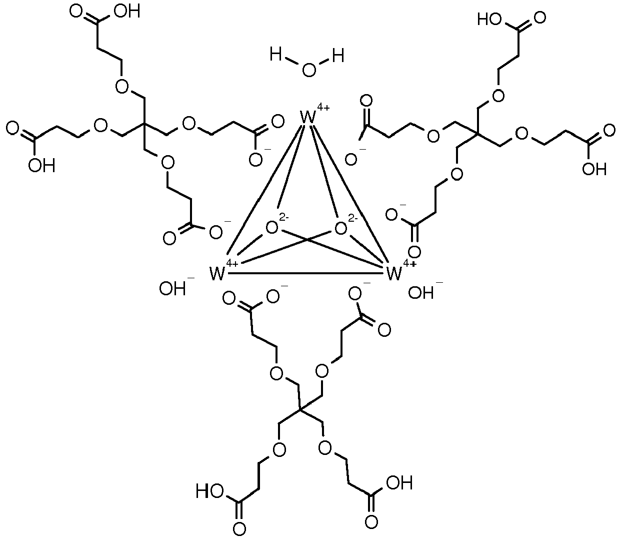

### Example 6a

### 3-{3-(2-Carboxyethoxy)-2,2-bis[(2-carboxyethoxy)methyl]propoxy}propanoic acid

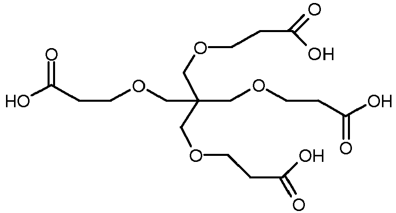

3-{3-(2-Carboxyethoxy)-2,2-bis[(2-carboxyethoxy)methyl]propoxy}propanoic acid was synthesized analogous to Weizman et al. J. Am. Chem. Soc. 1996, 118, 12368-12375.
**¹H-NMR** (400 MHz, D₂O) δ = 2.58 (t, 8 H), 3.34 (s, 8 H), 3.67 (t, 8 H).

### Example 6b

### Monoaqua-κO-tris{µ₂-3,3'-[1,3-bis(2-carboxyethoxy)propan-2,2-diyl]bismethyl (oxy)dipropanoato}1κ⁴O¹,O²,O³,O⁴:κ⁴O^{1'},O^{2'},O^{5'},O⁶:3κ²O^{3'},O^{4'},O^{5'},O^{6'}-dihydroxido-κ²O-di-µ3-oxido-1:2:3κ⁶O-triangu/o-tritungsten(IV)(3 W-W)

A suspension of 3-{3-(2-carboxyethoxy)-2,2-bis[(2-carboxyethoxy)methyl]propoxy}propanoic acid (0.47 g, 1.11mo) and monoaqua-κ*O*-hexakis(µ-acetato-κ²*O*)-dihydroxido-K²*O*-di-µ₃-oxido-1:2:3κ⁶*O*-*triangul*o-tritungsten (IV) (400 mg, 404 µmol) in acetic acid (140 µL, 2.4 mmol) and water (12 mL) was heated for 60 minutes to 120°C. The reaction mixtures were filtrated and the filtrates was concentrated in vacuum. Separation on a preparative HPLC (acetonitrile water + acetic acid) yielded 24 mg monoaqua-κ*O*-tris{µ₂-3,3'-[1,3-bis(2-carboxyethoxy)propan-2,2-diyl]bismethyl (oxy) dipropanoato} 1κ⁴*O*¹,*O*²,*O*³, *O*⁴:2κ⁴,*O*^{1'},*O*^{2'},*O*^{5'},*O*⁶:3κ²*O*^{3'}hydroxido-κ²*O*-di-µ₃-oxido-1:2:3κ⁶*O*-*triangulo-*tritungsten(IV)(3 W-W).
**¹H-NMR** (300 MHz, D₂O) δ = 2.57 (t, 12 H), 2.86 (br. t., 12 H), 3.31 (s, 12 H), 3.36 (s, 12 H), 3.69 (t, 12 H), 3.84 (br. t., 12 H) ppm.
**LC/MS ES-** m/z = 1901.13 (M-1).

### Example 7

### Monoaqua-κO-tris{µ-3,3'-[1-(2-carboxyethoxy)-3-methoxypropan-2,2-diyl]bismethyl (oxy)dipropanoato}1κ⁴O¹,O²,O⁴:2κ⁴O^{1'},O^{2'},O⁵,O⁶:3κ²O^{3'},O^{4'},O^{5'},O^{6'}-dihydroxido-κ²O-di-µ₃-oxido-1:2:3κ⁶O-triangulo-titungsten(IV)(3 W-W)

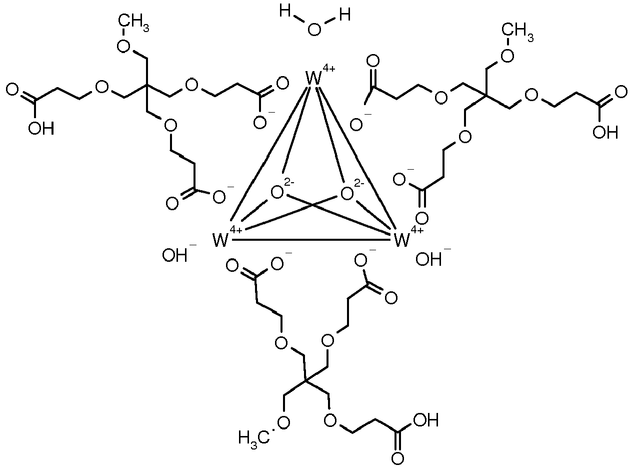

### Example 7a

### Tert-butyl 3-[3-(3-tert-butoxy-3-oxopropoxy)-2-[(3-tert-butoxy-3-oxopropoxy)methyl]-2-(methoxymethyl)propoxy]propanoate

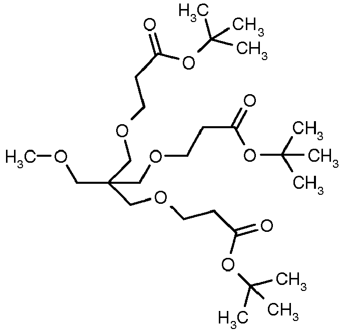

To 2-(hydroxymethyl)-2-(methoxymethyl)propane-1,3-diol (Journal of the American Chemical Society 1955, 77, 6382-3), (2.8 g, 20.2 mmol) in *tert*-butanol (15 mL) were added potassium *tert-*butoxide (0.68 g, 6 mmol) and *tert-*butyl acrylate (9.8 mL, 66.6 mmol). The suspension was stirred for 30 min at 100°C and overnight at room temperature. After removal of the solvent *in vacuo,* the residue was taken up in ethyl acetate and water, the organic phase was washed two times with water, dried over sodium sulfate. The mixture was concentrated *in vacuo* and the residue was purified by chromatography on silica gel (dichloromethane/methanol gradient, 0 to 100%) to yield 2.0 g (18.4 %) of *tert-*butyl 3-[3-(3-*tert*-butoxy-3-oxopropoxy)-2-[(3-*tert*-butoxy-3-oxopropoxy)-methyl]-2-(methoxymethyl)propoxy]propanoate.
**¹H-NMR** (400 MHz, CDCl₃): δ = 1.45 (s, 27H), 2.44 (t, 6H), 3.27 (s, 3H), 3.31 (s, 2H), 3.37 (s, 6H), 3.61 (t, 6H) ppm.
**ESI+** m/z = 535 (M+1).

### Example 7b

### 3-[3-(2-Carboxyethoxy)-2-[(2-carboxyethoxy)methyl]-2-(methoxymethyl)propoxy]-propanoic acid

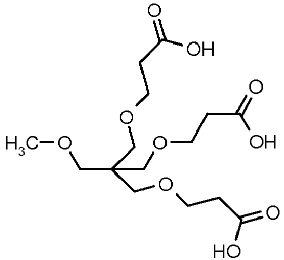

To a solution of *tert*-butyl 3-[3-(3-*tert*-butoxy-3-oxopropoxy)-2-[(3-*tert*-butoxy-3-oxopropoxy)methyl]-2-(methoxymethyl)propoxy]propanoate (1.34 g, 2.5 mmoL) in dioxane (20 mL) was added 2 molar aqueous hydrochloric acid (10 mL, 20 mmol). The mixture was refluxed for 6h and stirred at room temperature overnight. After removal of the solvent *in vacuo,* the residue was chromatographed with acetonitrile / water on C18-silica gel, the resulting fractions were combined and finally lyophilized to yield 0.9 g (98.2 %) of 3-[3-(2-carboxyethoxy)-2-[(2-carboxyethoxy)methyl]-2-(methoxymethyl)propoxy]propanoic acid.
**¹H-NMR** (400 MHz, D₂O) δ = 2.50 (t, 6H), 3.64 (s, 6H), 4.65 (m, 11H) ppm.
**ESI+** m/z = 367 (M+1).

### Example 7c

### Monoaqua-κO-tris{µ₃-3,3'-[1-(2-carboxyethoxy)-3-methoxypropan-2,2-diyl]bismethyl (oxy)dipropanoato}1κ⁴O¹,O²,O³,O⁴:2κ⁴O:2κ⁴O^{1'},O^{2'},O⁵,O⁶:3κ²O^{3'},O^{4'},O^{5'},O^{6'}-dihydroxido-κ²O-di-µ₃-oxido-1:2:3κ⁶O-triangulo-tritungsten(IV)(3 W-W)

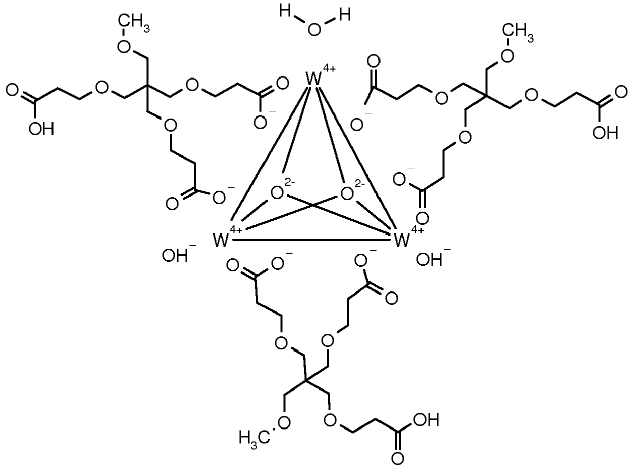

0.92 g (2.5 mmol) of 3-[3-(2-carboxyethoxy)-2-[(2-carboxyethoxy)methyl]-2-(methoxymethyl)propoxy]propanoic acid and 0.57 g, (0.5 mmol) of sodium hexakis(µ-acetato-κ²*O*)-tris(acetato-κ*O*)-di-µ₃-oxido-1:2:3κ⁶*O*-*triangulo*-tritungsten(3 *W-W*)(IV) in 40 mL water were irradiated in a micowave reactor for 8h at 125°C. The reaction mixture was filtrated and the solution was concentrated *in vacuo* and the resulting crude product was chromatographed with acetonitrile / water on C18-silica gel and the resulting fractions were combined and finally lyophilized. Monoaqua-κ*O*-tris{µ-3,3'-[1-(2-carboxyethoxy)-3-methoxypropan-2,2-diyl]bismethyl (oxy)dipropanoato}1κ⁴*O*¹,*O*³,*O*⁴:2κ⁴*O*^{1'},*O*^{2'},*O*⁵,*O*⁶:3κ² *O*^{3'},*O*^{4'},*O*^{6'}-dihydroxido-κ²*O-*di-µ₃-oxido-1:2:3κ⁶*O-triangulo*-tritungsten(IV)(3 W-W) was obtained (105 mg, 12.2 %) as a curry-colored amorphous powder.
**¹H-NMR** (600 MHz, D₂O) δ = 2.61 (t, 6H), 2.85 - 2.95 (m, 12H), 3.33 (s, 9H), 3.37 (s, 18H), 3.41 (s, 6H), 3.74 (t, 6H), 3.85 - 3.93 (m, 12H) ppm.
**LC/MS ES-** m/z = 1727.4 (M-1).

### Example 8

### Monoaqua-κO-tris{µ₃-3,3'-[1,3-dihydroxypropan-2,2-diyl]bismethyl (oxy)dipropanoato}1κ⁴O¹,O²O³,O⁴:2κ⁴O^{1'},O^{2'},O⁵,O⁶:3κ²O^{3'},O^{4'},O^{5'},O^{6'}-dihydroxido-κ²O-di-µ₃-oxido-1:2:3κ⁶O-triangulo-tritungsten(IV)(3 W-W)

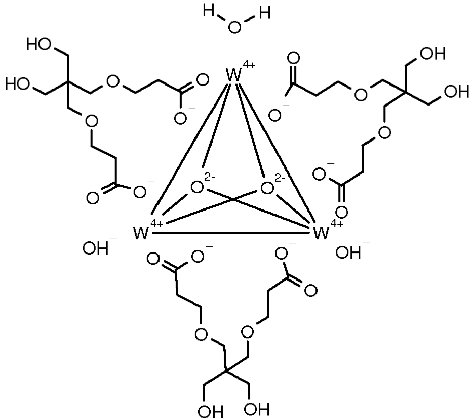

### Example 8a

### Di-tert-butyl 3,3'-{[2,2-bis(hydroxymethyl)propane-1,3-diyl]bis(oxy)}dipropanoate

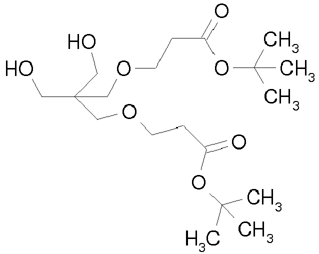

To pentaerythritol (40.0 g, 293.8 mmol) in *tert*-butanol (225 mL) was added potassium *tert-*butoxide (9.89 g, 88.14 mmol) and *tert-*butyl acrylate (142 mL, 969.5 mmol). The suspension was stirred for 40 min at 100°C and overnight at room temperature. After removal of the solvent *in vacuo,* the residue was taken up in ethyl acetate and water, the organic phase was washed two times with water and dried over sodium sulfate. The mixture was concentrated *in vacuo* and the residue was purified by chromatography on silica gel (dichloromethane/methanol gradient, 0 to 100%) to yield 30.3 g (26.3 %) of di-*tert-*butyl 3,3'-{[2,2-bis(hydroxymethyl)propane-1,3-diyl]bis(oxy)}dipropanoate.
**¹H-NMR** (500 MHz, DMSO-*d6*): δ = 1.40 (s, 18H), 2.38 (t, 4H), 3.28 (s, 3H), 3.33 (d, 4H), 3.52 (t, 4H), 4.10 (t, 2H) ppm.
**ESI+** m/z = 521 (M+1).

### Example 8b

### 3,3'-{[2,2-Bis(hydroxymethyl)propane-1,3.diyl]bis(oxy)}dipropanoic acid

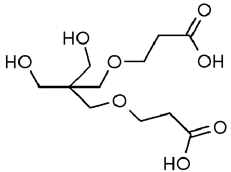

To a solution of di-*tert*-butyl 3,3'-{[2,2-bis(hydroxymethyl)propane-1,3-diyl]bis(oxy)}-dipropanoate (30.0 g, 76.4 mmoL) in dioxane (400 mL) was added 2 molar aqueous hydrochloric acid (200 mL, 400 mmol). The mixture was refluxed for 6h and stirred at room temperature overnight. After removal of the solvent *in vacuo,* the residue was chromatographed with acetonitrile / water on C18-silica gel, the resulting fractions were combined and finally lyophilized to yield 4.18 g (18.0 %) of 3,3'-{[2,2-bis(hydroxymethyl)propane-1,3-diyl]bis(oxy)}dipropanoic acid.
**¹H-NMR** (400 MHz, DMSO-*d6*) δ = 2.42 (t, 4H), 3.30 (s, 8H), 3.54 (t, 4H), 4.10 (br, 2H), 12.09 (br, 1H) ppm.
ESI+ m/z = 281 (M+1).

### Example 8c

### Monoaqua-κO-tris{µ-3,3'-[1,3-dihydroxypropan-2,2-diyl]bismethyl (oxy)dipropanoatol}1κ⁴O¹,O²,O³,O⁴:2κ⁴O^{1'},O^{2'},O⁵,O⁶:3κ²O^{3'},O^{4'},O^{6'}-dihydroxido-κ²O-di-µ₃-oxido-1:2:3κ⁶O-triangulo-tritungsten(IV)(3 W-W)

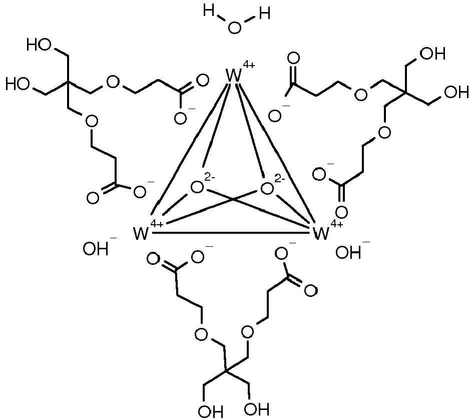

0.84 g (3 mmol) of 3,3'-{[2,2-bis(hydroxymethyl)propane-1,3-diyl]bis(oxy)}dipropanoic acid and 0.57 g, (0.5 mmol) of sodium hexakis(µ-acetato-κ²*O*)-tris(acetato-κ*O*)-di-µ₃-oxido-1:2:3κ⁶*O*-*triangulo*-tritungsten(3 *W-W*)(IV) in 19 mL water were irradiated in a micowave reactor for 3h at 125°C. The reaction mixture was filtrated and the solution was concentrated *in vacuo* and the resulting crude product was chromatographed with acetonitrile / water on C18-silica gel and the resulting fractions were combined and finally lyophilized. Monoaqua-κ*O*-tris{µ-3,3'-[1,3-dihydroxypropan-2,2-diyl]bismethyl (oxy)dipropanoato}1κ⁴*O*¹,*O*²,*O*⁴:2κ⁴*O*^{1'},*O*^{2'},*O*⁵,*O*⁶,3κ²*O*^{3'},*O*^{4'},*O*^{5'},*O*^{6'}-dihydroxido-κ²*O-*di-µ₃-oxido-1:2:3κ⁶*O*-*triangulo*-tritungsten(IV)(3 W-W) was obtained (80 mg, 10.9 %) as a curry-colored amorphous powder.
**¹H-NMR** (600 MHz, D₂O) δ = 2.74-2.83 (m, 8H), 2.90 (t, 4H), 3.27-3.33 (m, 8H), 3.40 (s, 4H), 3.40 (br. s., 4H), 3.51 (s, 4H), 3.54 (s, 4H), 3.76-3.82 (m, 4H), 3.87-3.94 (m, 8H) ppm.
**LC/MS ES-** m/z = 1469.30 (M-1).

### Example 9

### Monoaqua-κO-tris{µ-3,3'-[propane-1,3-diylbis(oxy)]dipropanoato}1κ⁴O¹,O²,O³,O⁴: 2κ⁴O^{1'},O^{2'},O^{5'},O⁶:3κ⁴O^{3'},O^{4'},O^{5'},O^{6'}-dihydroxido-κ²O-di-µ₃-oxido-1:2:3κ⁶O-triangulo-tritungsten(IV)(3 W-W)

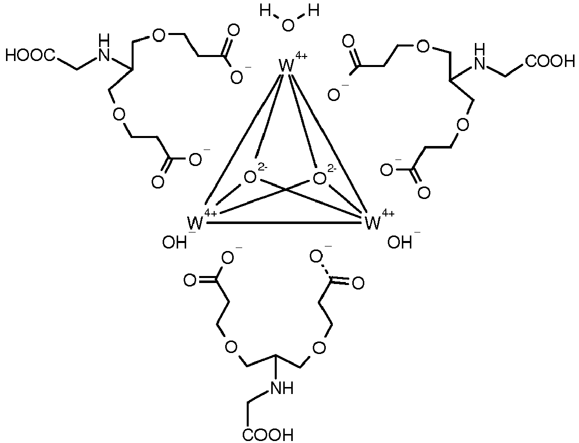

### Example 9 a

### Tert-butyl 3-({2-[(3-tert-butoxy-3-oxopropoxy)methyl]prop-2-en-1-yl}oxy)propanoate

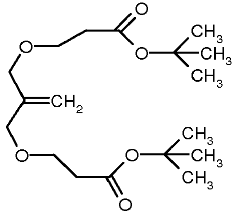

2-Methylidenepropane-1,3-diol (5.0 g, 56.7 mmol) and *tert-*butyl prop-2-enoate (36.4 g, 284 mmol) are stirred in DMSO (11.4 mL) and 5 molar aqueous sodium hydroxide solution (1.14 mL) at 20°C for 72 hours. The mixture was concentrated and the residue was purified by chromatography on silica gel (ethyl acetate in hexane, 0 to 40%) to yield 9.03 g of *tert*-butyl 3-({2-[(3-*tert*-butoxy-3-oxopropoxy)methyl]prop-2-en-1-yl}oxy)propanoate.
**¹H-NMR** (300 MHz, CDCl₃): δ = 1.46 (s, 18H), 2.50 (t, 4H), 3.66 (t, 4H), 3.98 (s, 4H), 5.18 (s, 2H) ppm.

### Examle 9b

### Di-tert-butyl 3,3'-[(2-oxypropane-1,3-diyl)bis(oxy)]dipropanoate

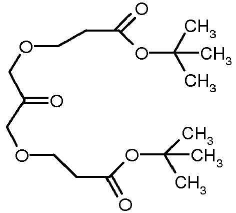

Into a solution of *tert-*butyl 3-({2-[(3-*tert*-butoxy-3-oxopropoxy)methyl]prop-2-en-1-yl}oxy)-propanoate (6.0 g, 17.4 mmol) in dichloromethane (180 mL) at -78°C was guided Ozone gas until a pale blue color was visible. The solution was stirred for 15 additional minutes at -78°C until dimethyl sulfide (6.4 mL, 87 mmol) was added and the reaction mixture was warmed to rt. Water was added and the mixture was extracted with dichloromethane. The combined organic phases were washed with brine, dried over sodium sulfate and concentrated to yield 6.0 g of *di-tert-*butyl 3,3'-[(2-oxypropane-1,3-diyl)bis(oxy)] dipropanoate.
**¹H-NMR** (400 MHz, CDCl₃): δ = 1.47 (s, 18H), 2.54 (t, 4H), 3.75 (t, 4H), 4.25 (s, 4H) ppm.

### Examle 9c

### Di-tert-butyl3,3'-[{2-[(2-tert-butoxy-2-oxoethyl)amino]propane-1,3-diyl}bis(oxy)]dipropanoate

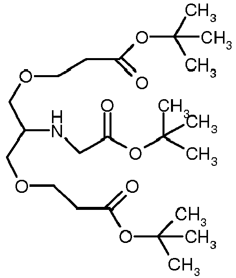

To a solution of di*-tert-*butyl 3,3'-[(2-oxypropane-1,3-diyl)bis(oxy)] dipropanoate (2.0 g, 5.77 mmol)and *tert-*butylgycinate (757 mg, 5.77 mmol) in methanol (80 mL) and acetic acid (15.6 mL) was added 5-ethyl-2-methylpyridine borane complex (0.34 mL, 2.3 mmol) at rt. After stirring for 20 hours the mixture was concentrated and the residue was purified by chromatography on silica gel (ethyl acetate in hexane, 10 to 60%) to yield 0.95 g of di-*tert-*butyl3,3'-[{2-[(2-*tert*-butoxy-2-oxoethyl)amino]propane-1,3-diyl}bis(oxy)]dipropanoate.
**¹H-NMR** (300 MHz, CDCl₃): δ = 1.45 (s, 18H), 1.46 (m, 9H), 2.49 (t, 4H), 2.57 (br. s., 1H), 2.94 (t, 1H), 3.34 - 3.51 (m, 4H), 3.39 (s, 2H), 3.66 (t, 4H) ppm.

### Examle 9 d

### 3,3'-[{2-[(Carboxymethyl)amino]propane-1,3-diyl}bis(oxy)]dipropanoic acid

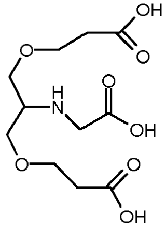

Di-*tert*-butyl3,3'-[{2-[(2-*tert*-butoxy-2-oxoethyl)amino]propane-1,3-diyl}bis(oxy)] dipropanoate (1.07 g, 2.33 mmol) was treated at 80°C with protonated ion exchange resin Amberlite IR 120 in dioxane (30 mL) and water (15 mL) for three hours. The ion exchange resin was packed into a column washed with water and eluted with 40 % acetic acid in water. The eluate was concentrated to yield 0.55 g of 3,3'-[{2-[(carboxymethyl)amino] propane-1,3-diyl}bis(oxy)]dipropanoic acid.
**¹H-NMR** (300 MHz, D₂O): δ = 2.66 (t, 4H), 3.70 (m, 1H), 3.72 (s, 2H), 3.74 - 3.78 (m, 3H), 3.78 - 3.84 (m, 5H) ppm.

### Example 9e

### Monoaqua-κO-tris{µ-3,3'-[2-({carboxymethyl}amino)propane-1,3-diylbis(oxy)] dipropanoato}1κ⁴O¹,O²,O³,O⁴,2κ⁴O^{1'},O^{2'},O⁵,O⁶:3κ⁴O^{3'},O^{4'},O^{5'},O^{6'},-dihydroxido-κ²O-di-µ3-oxido-1:2:3κ⁶O-triangulo-tritungsten(IV)(3 W-W)

A suspension of 3,3'-[{2-[(carboxymethyl)amino]propane-1,3-diyl}bis(oxy)]dipropanoic acid (550 mg, 1.88 mmol) and Sodium hexakis(µ-acetato-κ²*O*)-tris(acetato-κ*O*)-di-µ₃-oxido-1:2:3κ⁶*O*-*triangulo*-tritungsten(3 *W-W*)(IV) (696 mg, 0.63 mmol) in water (60 mL) was irradiated in a microwave reactor for 15 minutes at 140°C. The reaction mixtures were filtrated and the filtrate was concentrated in vacuum. Separation on a preparative HPLC (acetonitrile water + acetic acid ) yields 175 mg Monoaqua-κ*O*-bis{µ₃-3,3',3"-[ethyl-1,1,1-idynetris(methyleneoxy)]tripropanoato-1κ²*O*¹,*O*²:2κ²*O*^{1'},3κ²*O*^{2'},*O*^{3'}}dihydroxido-κ²*O*-di-µ₃-oxido-1:2:3κ⁶*O*-*triangulo*-tritungsten(IV)(3 W-W).
**¹H-NMR** (400 MHz, D₂O) δ = 2.68 - 2.98 (m, 12H), 3.40 - 3.50 (m, 4H), 3.55 - 3.65 (m, 4H), 3.63 (s, 4H), 3.68 - 3.79 (m, 12H), 3.84 - 4.08 (m, 10H) ppm.
**LC/MS ES-** m/z = 1506.6 (M-1).

### Example 10

### Stability of W₃O₂ Clusters

The stability of W₃O₂ clusters was determined in aqueous, buffered solution at pH 7.4. The solution containing 5 mmol/L of the compound in a tightly sealed vessel in which the air had been replaced by an argon or nitrogen atmosphere was heated to 121 °C for 45 min in a steam autoclave. In case of the formation of insoluble material, the precipitated material was removed by centrifugation. The tungsten concentration of the solution was determined by ICP-OES before and after heat treatment. The integrity of the compound was determined by HPLC analysis before and after heat treatment. Absolute stability was calculated as the ratio of the peak area of the compound after and before the heat treatment multiplied with the ratio of the tungsten concentration of the solution after and before heat treatment.

### HPLC system:

Column: Symmetry C18, 4.6 x 75 mm (Waters).
Solvent A1: 0.1 mM Na-citrate, pH 6-7
Solvent A2: 5 mM tetrabutylammonium phosphate pH 6
Solvent A3: 100 mM NH4-acetate pH 6-7
For selection of solvents A1 to A3 see the table below.
Solvent B: methanol, HPLC grade
Gradient: linear gradient starting from 100 % A and 0% B and ending at 5% A and 95% B after 10 min were used.
Flow: 1 mL/min
2 Detector were used in a serial connection: UV-vis, 200-600 nm, at 254 nm and specific absorption of W₃O₂-clusters at 460 nm. The UV-detector was connected to an ICP-MS running at m/z 184 for ¹⁸⁴W, the most abundant isotope of tungsten.

| | | Chromatographic conditions |
|---|---|---|
| Example No | Stability | Solvent A |
| 1 | 100 % | A2 |
| 2 | 100 % | A2 |
| 3 | 100 % | A2 |
| 6 | 98 % | A2 |
| 8 | 97 % | A1 |
| 9 | 73 % | A3 |

## Claims

1. Trinuclear tungsten clusters comprising bidentate di-, tri- or tetracarboxylic acid ligands.

2. Trinuclear tungsten cluster comprising three bidentate carboxylic acid ligands of the general formula I, 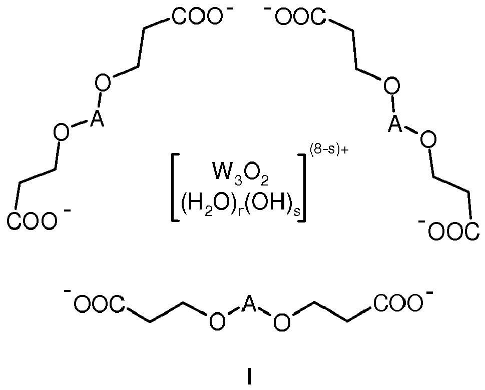 wherein
A is (CH₂)₂, (CH₂)₃, (CH₂)₄, (CH₂)₂-O-(CH₂)₂,CH₂-X-CH₂, (CH₂)₂-X-(CH₂)₂;
wherein X is CHOH, CHNH₂, C(CH₂OH)₂, C(CH₂OCH₃)₂, C(CH₂O(CH₂)₂COOH)₂,C(CH₂OH)(CH₂O(CH₂)₂COOH), C(CH₂OCH₃)(CH₂O(CH₂)₂COOH), CHNHCH₂COOH, CHNHCH₂CH₂OH, CHNH(CH₂)₂COOH, CHCH₂COOH, CH(CH₂)₂COOH;
r is 0, 1, 2, 3;
and
s is 0, 1, 2, 3;
with the proviso that r + s is 3;
if necessary any protonated species and any deprotonated species of said compounds, including all isomeric forms of said compounds, including but not limited to enantiomers, diastereomers, regioisomers and mixtures thereof, and any pharmaceutically acceptable salt of such compounds.

3. Trinuclear tungsten cluster of the general formula I:
Monoaqua-κ*O*-bis{µ₃-3,3'-[ethanediyibis(oxy)]dipropanoato-1κ*O*¹:2κ²*O*^{1'},*O*²:3κ*O*^{2'}}{µ-3,3'-[ethanediylbis(oxy)]dipropanoato}-1κ²*O*¹,*O*²:2κ²*O*^{1'},*O*^{2'}-dihydroxido-κ²*O-*di-µ₃-oxido-1:2:3κ⁶*O*-*triangulo*-tritungsten(IV)(3 W-W),
Monoaqua-κ*O*-tris{µ-3,3'-[ethanediyibis(oxy)]dipropanoato}-1κ⁴*O*¹,*O*²,*O*³,*O*⁴:2κ⁴*O*^{1'},*O*^{2'},*O*⁵,*O*⁶:3κ⁴*O*^{3'},*O*^{4'},*O*^{5'},*O*^{6'}-dihydroxido-κ²*O*-di-µ₃-oxido-1:2:3κ⁶*O-*triangulo-tritungsten(IV)(3 W-W),
Monoaqua-κ*O*-bis{µ₃-3,3'-[propane-1,3-diylbis(oxy)]dipropanoato-1*κ*:*O*¹:2κ²*O*^{1'},*O*²:3κ*O*²}{µ-3,3'-[propane-1,3-diyibis(oxy)]dipropanoato}-1*κ*²*O*¹,*O*²:2κ*O*^{1'},*O*^{2'}-dihydroxido-κ²*O*-di-µ₃-oxido-1:2:3κ⁶*O*-*triangulo-*tritungsten(IV)(3 W-W),
Monoaqua-κ*O*-tris(µ-3,3'-{oxybis[2,1-ethanediylbis(oxy)]}dipropanoato)-1κ⁴*O*¹,*O*²,*O*³,*O*⁴: 2κ⁴*O*^{1'},*O*^{2'},*O*⁵*,O*⁶:3κ⁴*O*^{3'}*,O*^{4'}*,O*^{5'},*O*^{6'}-dihydroxido-κ²*O*-di-µ₃-oxido-1:2:3κ⁶*O*-*triangulo-*tritungsten(IV)(3 W-W),
Monoaqua-κ*O*-bis(µ₃-3,3'-{oxybis[2,1-ethanediyibis(oxy)]}dipropanoato-1κ:*O*¹:2κ²*O*^{1'},*O*²: 3κ*O*^{2'})(µ-3,3'-{oxybis[2,1-ethanediylbis(oxy)]}dipropanoato)-1κ²*O*¹,*O*²:2κ²*O*^{1'},*O*^{2'}-dihydroxido-κ²*O*-di-µ₃-oxido-1:2:3κ⁶*O*-*triangulo*-tritungsten(IV)(3 W-W),
Monoaqua-κ*O*-tris{µ₂ -3,3'-[1,3-bis(2-carboxyethoxy)propan-2,2-diyl]bismethyl (oxy)dipropanoato}1κ⁴*O*¹,*O*²,*O*³,*O*⁴:2κ⁴*O*^{1'},*O*^{2'},*O*⁵,*O*⁶:3κ²*O*^{3'},*O*^{4'},*O*^{5'},*O*^{6'}dihydroxido-κ²-*O*-di-µ₃-oxido-1:2:3κ⁶*O*-*triangulo*-tritungsten(IV)(3 W-W),
Monoaqua-κ*O*-tris{µ-3,3'-[1-(2-carboxyethoxy)-3-methoxypropan-2,2-diyl]bismethyl (oxy)dipropanoato}1κ⁴*O*¹,*O*²,*O*³,*O*⁴:2κ⁴*O*^{1'},*O*^{2'},*O*⁵,*O*⁶:3κ²*O*^{3'},*O*^{4'},*O*^{5'},*O*^{6'}dihydroxido-κ²*O*-di-µ₃-oxido-1:2:3κ⁶*O*-triangulo-tritungsten(IV)(3 W- W),
Monoaqua-κ*O*-tris{µ-3,3'-[1,3-dihydroxypropan-2,2-diyl]bismethyl (oxy)dipropanoato}1κ⁴*O*¹,*O*²,*O*³,*O*⁴:2κ⁴*O*^{1'},*O*^{2'},*O*⁵,*O*⁶:3κ²*O*^{3'},*O*^{4'},*O*^{5'},*O*^{6'}dihydroxido-κ²*O*-di-µ₃-oxido-1:2:3κ⁶*O*-*triangulo*-tritungsten(IV)(3 W- W), and
Monoaqua-κ*O*-tris{µ-3,3'-[2-({carboxymethyl}amino)propane-1,3-diyibis(oxy)]dipropanoato}-1κ⁴*O*¹,*O*²,*O*³,*O*⁴:2κ⁴*O*^{1'},*O*^{2'},*O*⁵,*O*⁶:3κ²*O*^{3'},*O*^{4'},*O*^{5'},*O*^{6'}-dihydroxido-κ²*O*-di-µ₃-oxido-1:2:3κ⁶*O*-triangulo-tritungsten(IV)(3 W-W),
and if necessary protonated species and deprotonated species of said compounds and suitable salts thereof or regioisomers thereof.

4. Trinuclear tungsten clusters comprising three bidentate carboxylic acid ligands of general formulae I of claim 2, obtainable by ligand exchange reaction of monoaqua-κ*O-*hexakis(µ-acetato-κ²*O*)-dihydroxido-κ²*O*-di-µ₃-oxido-1:2:3K⁶*O*-triangulo-tritungsten(3 *W-W*)(IV) or a salt of this cluster or sodium hexakis(µ-acetato-κ²*O*)-tris(acetato-O)-di-µ₃-oxido-1:2:3K⁶*O*-triangulo-tritungsten(3 *W-W*)(IV) in aqueous solution in the presence of three aliquots or slight excess of the desired bidentate ligand, heating the components to temperatures in the range from 80° to 150°C in combination with the optional use of a pressure vessel, if necessary applying microwave irradiation, applying heating times range from 10 minutes up to 3 days, isolation and purification with chromatography or ion exchange chromatography.

5. Process for the preparation trinuclear tungsten clusters comprising three bidentate carboxylic acid ligands of general formula I of claim 2, by reaction of monoaqua-κ*O*-hexakis(µ-acetato-κ²*O*)-dihydroxido-κ²*O*-di-µ₃-oxido-1:2:3K⁶*O*-triangulo-tritungsten(3 *W-W*)(IV) or a salt of this cluster or sodium hexakis(µ-acetato-κ²*O*)-tris(acetato-κ*O*)-di-µ₃-oxido-1:2:3κ⁶*O*-*triangulo*-tritungsten(3 *W-W*)(IV) in aqueous solution in the presence of three aliquots or slight excess of the desired bidentate ligand, heating by temperatures in the range from 80° to 150°C in combination with the optional use of a pressure vessel, if necessary applying microwave irradiation, isolation and purification with chromatography or ion exchange chromatography.

6. Use of the compounds of general formula I for the manufacture of diagnostic agents, especially X-ray diagnostic agents.
